(19) Europäisches Patentamt

European Patent Office

Office européen des brevets

(11) **EP 0 709 087 B1**

(12) **FASCICULE DE BREVET EUROPEEN**

(45) Date de publication et mention
de la délivrance du brevet:
**29.12.1999 Bulletin 1999/52**

(51) Int. Cl.[6]: **A61K 9/50**

(21) Numéro de dépôt: **95420286.7**

(22) Date de dépôt: **18.10.1995**

(54) **Microcapsules médicamenteuses et/ou nutritionnelles pour administration per os**

Arzneimittel und/oder Nahrungsmikrokapseln zur oralen Verabreichung

Medicinal and/or nutritional microcapsules for oral administration

(84) Etats contractants désignés:
**AT BE CH DE DK ES FR GB GR IE IT LI LU MC NL PT SE**
Etats d'extension désignés:
**LT LV SI**

(30) Priorité: **18.10.1994 FR 9412759**

(43) Date de publication de la demande:
**01.05.1996 Bulletin 1996/18**

(73) Titulaire:
**FLAMEL TECHNOLOGIES, Société Anonyme
69693 Venissieux Cédex (FR)**

(72) Inventeurs:
• **Autant, Pierre
F-03600 Commentry (FR)**
• **Selles, Jean-Philippe
F-34000 Montpellier (FR)**
• **Soula, Gérard
F-39330 Meyzieu (FR)**

(74) Mandataire:
**Fleurance, Raphael et al
Cabinet Plasseraud
Le Danica B
21, avenue G. Pompidou
69003 Lyon (FR)**

(56) Documents cités:
**EP-A- 0 207 041            DE-A- 3 943 242**

EP 0 709 087 B1

## Description

Le domaine de la présente invention est celui des systèmes à libération prolongée de principes actifs médicamenteux et/ou nutritionnels (PA), destinés à une administration par la voie orale.

La présente invention concerne ainsi des microcapsules destinées à l'administration per os, et contenant au moins un PA, à l'exclusion de l'acide acétylsalicylique, et qui présentent, notamment, comme caractéristique essentielle un temps de résidence dans l'intestin grêle supérieur à la durée du transit naturel, ce qui permet ainsi une augmentation de la durée d'absorption effective in vivo de ces PA.

L'invention concerne également un procédé de préparation des microcapsules évoquées ci-dessus.

Les systèmes galéniques plus particulièrement concernés par la présente invention sont ceux du type permettant une absorption prolongée (voire contrôlée) dans l'intestin grêle. La présente invention n'exclut pas l'utilisation des PA présentant une éventuelle absorption non spécifique au niveau de l'estomac et/ou du côlon.

L'intérêt des systèmes à libération prolongée pour l'administration d'un médicament est bien connu. Ils permettent en particulier de mieux assurer la couverture du besoin thérapeutique, car la concentration plasmatique utile en PA peut être maintenue plus longtemps que dans le cas des formes à libération instantanée. De plus ils permettent d'éviter, ou de limiter, l'importance et le nombre des pics de concentration excessive en PA au niveau plasmatique, ce qui diminue la toxicité du médicament et ses effets secondaires. Par ailleurs, ces systèmes permettent, par leur durée d'action accrue, de limiter le nombre de prises quotidiennes, ce qui diminue la contrainte pour le patient et améliore l'observance du traitement.

Il a ainsi été recherché des systèmes permettant de prolonger l'action d'un médicament, et les références concernant cet objectif sont nombreuses. On consultera à cet égard avec intérêt, Formes Pharmaceutiques Nouvelles, BURI, PUISIEUX, DOELKER et BENOIT, Lavoisier 1985, p175-227.

L'art antérieur décrit, par exemple, des tentatives visant à réaliser des systèmes à libération prolongée, avec l'objectif de fournir des formes médicamenteuses par exemple à prise journalière unique.

Il a ainsi été proposé des systèmes monolithiques, comme des comprimés, dans lesquels la dose à administrer est présentée sous la forme d'un objet massif.

Ainsi, le DE-A-39 43 242 (FR-A-2 670 112) divulgue des granulés du type matriciel, comprenant des particules de PA, éventuellement additionné d'excipient(s) inerte(s). Chaque granulé est formé par une multitude de particules incluses dans une matrice approximativement sphérique, comprenant un polymère cellulosique, un polymère vinylique, un plastifiant et un lubrifiant.

Ces granulés sont des formes pharmaceutiques définies par les pharmacopées. Elles se distinguent nettement des microcapsules qui sont elles aussi définies dans les pharmacopées. Ces granulés sont, par ailleurs, destinés à faire des comprimés monolithiques dont les inconvénients seront évoqués ci-dessous. En outre, ces granulés comprimables ont une taille supérieure ou égale à 1,2 mm.

La matrice des granulés décrits dans les exemples de cette demande DE 39 43 242 comprend toujours un polyacrylate et un polymère cellulosique, ce dernier étant présent dans une faible proportion, inférieure ou égale à 50 % en poids sur sec par rapport à l'ensemble des constituants de la matrice.

En outre, cette demande enseigne que, pour obtenir des caractéristiques de bioadhésivité, la matrice des granulés doit contenir les polymères acrylate et cellulosique dans un rapport nettement favorable au polyacrylate.

En tout état de cause, il n'y a aucune raison pour que ces granulés séjournent dans l'intestin grêle pendant une durée supérieure à la durée du transit gastrointestinal naturel. D'ailleurs, cette caractéristique n'est nullement décrite ni même suggérée dans cette demande DE.

On connaît, également, de nombreux comprimés pelliculés à l'aide d'un matériau d'enrobage par exemple de type cellulosique, acrylique, d'amidons, de polyéthylène glycol, de gommes ou analogues de ces produits. Cet enrobage permet à la fois aux comprimés de résister aux fluides physiologiques et par là même, de protéger les PA sensibles dans ces milieux pour augmenter, in fine, la biodisponibilité des PA, mais également de prolonger la libération desdits PA.

Ainsi, le brevet US 4 461 759 décrit un comprimé enrobé préservant le PA des effets nocifs de l'acidité de l'estomac et ayant la propriété supposée de libérer le PA à vitesse constante dans le tractus gastrointestinal. En effet, aucune analyse de déconvolution n'ayant été réalisée, cette propriété n'est pas établie.

Un autre exemple de forme comprimée monolithique est celle consistant à mettre en oeuvre un pelliculage microporeux permettant la libération du PA sous l'effet d'une pression osmotique. Cette libération prolongée s'effectue quelle que soit la solubilité du PA dans le milieu. Cette forme de réalisation est décrite dans la demande de brevet WO 91/16 885 et dans le brevet US 5 028 434.

Ces systèmes monolithiques pelliculés présentent des possibilités d'utilisation limitées pour plusieurs raisons. Tout d'abord, dans ces systèmes, la dose de médicament se présente en une seule entité physique, ce qui présente le risque de libération d'une quantité massive de PA, soit par mastication au moment de la prise, soit par rupture du pelliculage pendant le transit gastro-intestinal, ce qui perturbe leur efficacité thérapeutique et présente des risques d'effets secondaires graves. De plus, compte tenu de leur taille importante, ces systèmes ne peuvent sortir de l'estomac que lorsque le pylore est ouvert. Or l'ouverture du pylore se fait de manière séquentielle et en fonction de l'alimentation. En conséquence, le temps de séjour dans l'estomac des systèmes monolithiques varie énormément en fonction de l'heure, du volume et de la nature des repas, mais également selon l'individu. Ces systèmes présentent donc une grande variabilité d'absorption, voire de biodisponibilité, en fonction de l'individu et du moment de la prise. D'autre part, leur temps de résidence dans l'intestin grêle est soumis au transit naturel, et ces formes se retrouvent rapidement dans le côlon où s'achève leur libération. Or, l'absorption côlonique est faible pour un grand nombre de PA. De plus elle est très irrégulière étant donné la forte viscosité du milieu, la faible surface de la muqueuse côlonique, et la grande variabilité du temps de transit à ce niveau. Ainsi, pour ces systèmes, il est bien connu que la libération prolongée n'est pas synonyme d'une absorption prolongée au-delà du temps de transit dans l'intestin grêle.

Afin d'éviter les écueils inhérents aux systèmes monolithiques, il a été proposé des formes multiparticulaires dans lesquelles les éléments constitutifs ont une taille moyenne individuelle généralement inférieure à 2 mm. Cette taille permet le franchissement de l'estomac indépendamment de l'ouverture du pylore. Le temps de transit gastrique est ainsi plus rapide et surtout plus régulier. Dans la pratique, ces formes multiparticulaires sont essentiellement administrées sous forme de gélules ou de comprimés rapidement délitables.

La littérature technique antérieure ne manque pas de descriptions de systèmes galéniques microparticulaires à libération prolongée de PA.

Par exemple, le brevet EP 0 396 425 divulgue un système destiné à l'administration en une dose journalière unique de PA comme des nitrates, des éphédrines et du chlorure de potassium. A cette fin, le PA est fixé à la surface de sphérules inertes présentant un diamètre allant de 250 à 2 000 microns, au moyen d'un agent liant connu. Les particules sont ensuite pelliculées par un composé cellulosique et un plastifiant, destinés à ralentir la libération du PA. Ces particules pelliculées sont utilisées généralement en mélange avec des particules non enrobées destinées à fournir une dose initiale immédiatement libérée dans l'organisme. Les essais de dissolution in vitro montrent que le PA est libéré en 24 heures environ, mais aucune mesure de biodisponibilité n'est réalisée in vivo. Cette demande ne fait pas mention d'un allongement du temps de transit et d'absorption in vivo dans l'intestion grêle.

Le brevet US 5 286 497 décrit une formulation à base de Diltiazem (PA) conçue pour une prise journalière unique. A cette fin, le système est obtenu par le mélange de deux types de particules renfermant le Diltiazem. Le PA est fixé à la surface de granulés inertes en sucre ou en amidon, qui sont ensuite éventuellement pelliculés. Il présente une teneur maximale en Diltiazem de l'ordre de 45 % du poids de la forme finale. Dans cette forme médicamenteuse, un premier type de particules présente une libération rapide et assure, dans un premier temps, la couverture thérapeutique, tandis que le second type est à action retardée et ne commence sa propre libération qu'en fin d'action des particules du premier type. Outre sa faible teneur en PA, ce système présente l'inconvénient de nécessiter la préparation et le mélange de deux types de particules, ce qui complique le procédé de fabrication et grève son coût de revient. Par ailleurs, ce système qui fournit deux doses successives de PA est adapté au cas particulier du Diltiazem. Ce produit est un PA qui présente une dégradation importante lors du premier passage hépatique. En outre, ce brevet ne fait pas mention d'un temps prolongé de résidence et d'absorption in vivo au niveau de l'intestin grêle et les particules du système objet de ce brevet sont soumises au transit gastrointestinal naturel. De plus, la dose contenue dans les particules de 2ème type à action retardée, et qui commencent leur libération environ 12 heures après l'ingestion, est délivrée et absorbée au niveau côlonique. Enfin, la dose contenue dans les particules du premier type est à libération rapide et peut induire des pics plasmatiques préjudiciables à une bonne tolérance.

Le Diltiazem présente par ailleurs une durée de demi-vie dans l'organisme importante, de l'ordre de 6 heures. Dans ce cas, la concentration plasmatique se maintient naturellement au-dessus du seuil d'efficacité, pendant un temps suffisamment long pour permettre la réalisation aisée d'une forme à prise journalière unique. C'est ce qu'exploite l'EP 0 315 197 qui décrit également une forme de Diltiazem à prise journalière unique, capable de maintenir le taux plasmatique pendant 24 heures, au moyen d'un comprimé libérant 90 % d'une dose comprise entre 90 et 270 mg, en 5 heures.

Lorsque le PA présente une grande vitesse d'absorption, ou une lente vitesse d'élimination biologique, sa demi-vie plasmatique est naturellement longue, et la préparation d'un médicament à action prolongée est aisée. Mais il n'en va pas de même pour la préparation d'un médicament à base d'un PA à demi-vie plasmatique courte, par exemple inférieure à 3 heures. En effet, un tel PA doit être mis à disposition dans l'organisme au fur et à mesure de son utilisation.

En conséquence, le faible temps de séjour dans l'intestin grêle, pose un important problème à l'homme de l'art s'intéressant à la mise au point de médicaments à absorption prolongée, destinés à une administration par voie orale. Ce problème n'a pas été résolu à ce jour. Le médicament administré par voie orale est en effet soumis au transit naturel du tractus gastro-intestinal, ce qui limite son temps de résidence. Or l'intestin grêle est le siège privilégié de l'absorption systémique et il représente le site idéal pour la mise à disposition des PA. Ainsi, on comprend bien l'intérêt d'une forme galénique présentant un temps de séjour accru dans l'intestin grêle, afin d'assurer une absorption prolongée du PA in vivo, au-delà des durées normales de transit dans l'intestin grêle.

De nombreuses études ont été réalisées concernant le temps de transit gastrointestinal. Ces études montrent que la durée du transit gastrique est très variable notamment en fonction de l'alimentation, et qu'elle est comprise entre quelques minutes et quelques heures. En revanche, au niveau de l'intestin grêle, la durée de transit est particulièrement constante et plus précisément de 3 heures plus ou moins une heure (voir par exemple S.S. DAVIS : Assessment of gastrointestinal transit and drug absorption, in Novel drug delivery and its therapeutic application, Ed L.F. PRESCOTT- W.S. NIMMO. 1989. J. WILEY & SON, p. 89-101).

Or, comme indiqué supra, il serait intéressant, dans un grand nombre de cas, de pouvoir délivrer les PA au niveau de l'intestin grêle qui est le siège privilégié de l'absorption systémique. La durée de résidence idéale pour un système à libération prolongée s'adressant au plus grand nombre de PA, y compris ceux présentant une demi-vie plasmatique très courte, serait en conséquence supérieure à 5 heures, de préférence à 7 heures, et par exemple comprise entre 8 et 24 heures, ce qui permet de couvrir l'ensemble du nychtémère.

L'intérêt d'un système présentant un temps de résidence dans l'intestin grêle de l'ordre de 8 à 24 heures, et assurant ainsi une libération et une absorption prolongées d'au moins 90 % de la dose durant tout ou partie de cette période, serait de plusieurs ordres :

- tout d'abord, en assurant la libération d'une dose du PA à un flux optimal, il permettrait d'en améliorer la biodisponibilité et de limiter la dose à administrer,
- de plus, il permettrait l'obtention de taux plasmatiques prolongés pour les PA qui présentent une durée de demi-vie plasmatique courte ; ceci permettrait de limiter au mieux le nombre de prises quotidiennes et, en particulier, la préparation de systèmes à prise journalière unique pour un plus grand nombre de PA ; la réalisation de telles formes ne serait alors limitée que par le volume de la dose à administrer, qui doît rester acceptable pour le patient.

Des formes galéniques à administration par voie orale ont été réalisées avec l'intention d'augmenter artificiellement leur temps de résidence dans le tractus gastrointestinal. De telles formes ont pour objectif un transit propre, indépendant du transit naturel.

La littérature décrit en particulier des comprimés dits "flottants", caractérisés par un temps de résidence important dans l'estomac. Par exemple, le brevet US 4 869 908 décrit un tel système. Ce système est plus particulièrement adapté à l'administration des PA présentant une absorption préférentielle au niveau gastrique, ce qui est très limitatif.

D'autres travaux ont aussi été réalisés avec le même objectif d'accroissement du temps de transit, mais pour des systèmes microparticulaires.

Le brevet FR 2 395 026 revendique un procédé de préparation d'un système où les microparticules contenant le PA sous une forme à libération prolongée, renferment dans leur composition un agent densifiant, permettant un allongement significatif du temps de transit pouvant alors dépasser 24 heures. Ce système a été mis au point après constatation du fait que le transit dans l'intestin grêle est notablement ralenti lorsque la masse spécifique des particules dépasse 1,4 gramme par centimètre cube. La même approche d'accroissement du temps de transit par élévation de la masse spécifique est retenue dans les demandes EP 0 080 341 et EP 0 173 210. De tels systèmes présentent néanmoins l'inconvénient de nécessiter l'introduction d'une proportion importante d'agent densifiant, de l'ordre de 30 à 80 % du poids total de la forme, ce qui limite la teneur en PA du système et constitue un handicap pour la fabrication de formes nécessitant une dose importante de PA.

Une autre approche de l'allongement du temps de transit consiste en la mise au point de systèmes bioadhésifs.

Ainsi, la demande EP 0 452 268 revendique un système buccoadhésif sous la forme de microparticules pelliculées par un gel de gommes xanthane/caroube ou par de l'éthylcellulose. L'efficacité d'un tel système, essentiellement destiné à la cavité buccale, n'est pas établie, d'autant plus que les particules sont recouvertes en couche externe, d'une pellicule de cire, destinée à prolonger leur libération, mais qui rend l'adhésion improbable et, en tous cas, non démontrée in vivo.

La demande EP 0 516 141 vise à la mise au point d'un système particulaire bioadhésif par surenrobage, d'une forme quelconque à libération prolongée d'un PA, par une composition adhésive à base de polymères comme des dérivés hydrosolubles de la cellulose, des polymères acryliques connus sous les marques Carbopol® ou Polycarbophil®, des alginates, de la gélatine ou la pectine. Cette invention prétend ainsi séparer dans les systèmes en question deux fonctions essentielles, qui sont le contrôle de la libération du PA, d'une part, et la bioadhésion d'autre part. Les essais de validation d'une telle forme se limitent, cependant, à des tests ex vivo et n'ont pas prouvé une absorption prolongée in vivo.

De nombreux auteurs ont étudié des substances potentiellement bioadhésives, comme le carboxyméthylcellulose, l'acide polyacrylique, le Carbopol®, le Polycarbophil®, la gélatine et autres polymères naturels ou synthétiques. Ces substances sont par exemple décrites et évaluées par D. DUCHENE et al. - Pharmaceutical and medical aspects ofbioadhesive systems for drug administration, in Drug. Dev. Ind. Pharm.14(2&3), 283-318(1988) et J.M. GU et al. - Binding of acrylic polymers to mucin/epithelial surfaces, structure-property relationships, in CRC Crit. Rev. in Therap. Drug. Carrier Syst, Vol.5, Issue 1 (1988). Il a été trouvé que certains polymères présentent des propriétés adhésives au niveau de certaines muqueuses, orale ou vaginale par exemple. Mais de façon générale, aucune bioadhésion n'est établie in vivo au niveau de l'intestin grêle, pour ces substances, et/ou pour les formes à administration par voie orale existantes. Aucune preuve de l'adhésion dans l'intestin grêle n'est en effet apportée, ni par observation directe, ni par une étude de pharmacocinétique établissant un temps de résidence prolongé à ce niveau, objectivé par une absorption in vivo prolongée. A ce propos, on consultera par exemple A.J. MOËS - Gastroretentive dosage forms, in Crit. Rev. Ther Drug Carrier Syst. 10 (2) 143-195 (1993) et A.T. FLORENCE - Drug Delivery : Advances and Commercial Opportunities in Connect Pharma LTD p 40-44.

On doit cependant noter que les polymères présentant, et de loin, la meilleure bioadhésion selon les tests in vitro ou ex vivo décrits dans la littérature, sont essentiellement les dérivés des acides acryliques et méthacryliques.

La revue de l'art antérieur fait apparaître un grand nombre de tentatives vaines, visant à fournir une solution générale à la rétention et libération prolongées de PA dans l'intestin grêle, pendant des durées pouvant atteindre 24 heures dans le cadre d'administrations per os. De plus, aucune ne prend en compte l'ensemble des contraintes inhérentes à la réalisation d'un système multifonctionnel applicable au plus grand nombre de PA et aucune solution satisfaisante n'est disponible à ce jour.

Il existe, en effet un grand nombre de contraintes s'opposant à la réalisation d'un tel système et les difficultés à résoudre sont nombreuses.

Certaines de ces contraintes et difficultés sont énumérées ci-après :

- il est intéressant que le système présente un transit rapide et régulier dans l'estomac, afin d'assurer la reproductibilité de l'effet thérapeutique intra et inter individu,
- il est avantageux que le système reste présent dans l'intestin grêle pendant toute la durée nécessaire à l'absorption de la dose, en particulier pour les PA présentant une durée de demi-vie d'élimination ; il est ainsi préférable que le système réside dans l'intestin grêle pendant une durée notablement supérieure au temps de transit naturel,
- il est préférable que le système puisse présenter une forte teneur en PA afin de permettre la réalisation du médicament, même lorsque la dose de PA est importante, tout en respectant le confort du patient,
- il est préférable que le système permette d'éviter le risque d'une absorption massive dans l'organisme de l'ensemble, ou d'une fraction importante, de la dose destinée normalement à la couverture d'une longue période,
- il est souhaitable que le système ne permette pas la libération d'une quantité importante de PA, pendant une durée prolongée à un niveau localisé de la muqueuse gastrointestinale, afin d'éviter les risques d'ulcération,
- il est préférable que le système présente une résistance mécanique suffisante pour permettre l'absorption progressive du PA, selon un profil déterminé et reproductible in vivo, et ce jusqu'à épuisement de la dose,
- il est souhaitable que le système protège au mieux le PA contre les éventuelles agressions des milieux physiologiques pendant sa durée de résidence dans l'organisme,
- il est avantageux que le système soit insensible aux variations du pH rencontrées sur l'ensemble du tractus gastrointestinal, afin de préserver la régularité de la mise à disposition du PA,
- enfin il est souhaitable qu'un tel système puisse être fabriqué de façon simple et économique.

Dans ce contexte, force est de constater qu'il existe une carence en un système galénique pour l'administration de PA par voie orale, qui possède, cumulativement et pour une large gamme de PA, les spécifications suivantes entre autres :

- transit rapide et régulier dans l'estomac, reflété par l'absence d'un temps de latence du profil d'absorption in vivo indépendant de l'activité gastrique,
- transit lent dans l'intestin grêle, reflété par un profil d'absorption in vivo sur une durée notablement supérieure à celle permise par le transit naturel (3 h ± 1),
- absorption progressive du PA et ce jusqu'à épuisement de la dose (supérieure à 90 %)
- absence d'irritation de la muqueuse,
- teneur en PA élevée,
- coût de revient réduit.

L'un des objectif essentiels de la présente invention est de pallier cette carence.

A cette fin, la présente invention a pour objet un système galénique formé par des microcapsules, de type réservoir, contenant au moins un Principe Actif médicamenteux et/ou nutritionnel (PA), à l'exclusion de l'acide acétylsalicylique (ASA), destinées à l'administration par voie orale, caractérisées :

- en ce qu'elles sont constituées par des particules de PA recouvertes chacune d'une pellicule d'enrobage de composition suivante :

  1 - au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, l'éthylcellulose et/ou l'acétate de cellulose étant particulièrement préférés ;

2 - au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N--vinylamide et/ou un polyN-vinyl-lactame, le polyacrylamide et/ou la polyvinylpyrrolidone étant particulièrement préférés ;

3 - au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin, l'acide salicylique et la cutine, l'huile de ricin étant particulièrement préférée;

4 - au moins un agent tensio-actif et/ou lubrifiant, présent à raison de 2 à 20, de préférence de 4 à 15 % en poids sur sec par rapport à la masse totale de la composition d'enrobage et choisi parmi les tensio-actifs anioniques, de préférence les sels alcalins ou alcalinoterreux des acides gras, l'acide stéarique et/ou oléique étant préférés, et/ou parmi les tensio-actifs non ioniques, de préférence les esters de sorbitan polyoxyéthylénés et/ou les dérivés de l'huile de ricin polyoxyéthylénés, et/ou parmi les agents lubrifiants comme les stéarates, de préférence de calcium, de magnésium, d'aluminium ou de zinc, ou comme le stéarylfumarate, de préférence de sodium, et/ou le béhénate de glycérol ; ledit agent pouvant comprendre un seul ou un mélange des susdits produits;

- en ce qu'elles possèdent une granulométrie comprise entre 50 et 1 000 microns, de préférence entre 100 et 750 microns, et, plus préférentiellement encore, entre 100 et 500 microns,
- en ce qu'elles sont conçues de manière à pouvoir séjourner dans l'intestin grêle pendant un temps d'au moins 5 heures environ, de préférence au moins 7 heures environ et, plus préférentiellement encore, pendant un temps compris entre environ 8 heures et environ 24 heures et permettre ainsi l'absorption du PA pendant au moins une partie de leur temps de séjour dans l'intestin grêle.

Il est du mérite de la Demanderesse, d'avoir mis au point de façon tout à fait surprenante et inattendue, un tel système galénique.

La présente invention divulgue ainsi un nouveau système à libération prolongée qui présente entre autres caractéristiques essentielles et simultanées, un temps de séjour réduit dans l'estomac et une durée de transit dans l'intestin grêle notablement supérieure à la durée du transit naturel. En particulier, le système à absorption prolongée selon l'invention présente un temps de résidence dans l'intestin grêle, compris entre 5 et 24 heures, c'est à dire de 2 à 12 fois supérieur au temps de transit naturel. L'absorption du PA est naturellement liée à sa libération hors des microcapsules.

Pour un PA donné, un temps de résidence prolongé peut être mis en évidence par la mesure d'une durée d'absorption in vivo, excédant largement la durée du transit naturel dans l'intestin grêle. Cette mesure de la durée d'absorption est obtenue au travers de la concentration plasmatique du PA et constitue un moyen fiable et reconnu d'objectivation du temps de résidence.

Le système revendiqué permet également, et ceci indépendamment de ce temps de séjour important, d'assurer la libération prolongée des PA mis en oeuvre selon la présente invention. Il convient de noter que la présence du système pendant une durée de l'ordre de 24 heures dans l'intestin grêle, n'impose en aucune façon une libération continue sur l'ensemble de cette période. L'homme de l'art saura modifier la durée de libération dans un intervalle de temps adapté au PA particulier considéré, en fonction de l'objectif pharmacologique visé.

La présente invention donne notamment accès à des formes pharmaceutiques, per os, à doses journalières uniques pour un plus grand nombre de PA que les formes pharmaceutiques selon l'art antérieur.

Dans l'exposé de l'invention, il sera fait appel au terme de "microcapsules" qui désigne les particules pelliculées et les distingue des particules de PA non pelliculées, qui seront appelées "microparticules".

Avantageusement, la pellicule d'enrobage présente une résistance mécanique suffisante pour éviter son déchirement et/ou son éclatement dans l'organisme et ce jusqu'à la fin de la libération du principe actif. Cette aptitude de la pellicule à conserver son intégrité physique même après élution complète du PA s'observe, notamment, pour des épaisseurs d'enrobage comprises entre 2 et 100 microns.

Ces microcapsules peuvent être assimilées à des véhicules permettant le transport et la libération d'un ou plusieurs PA dans l'intestin grêle.

De préférence, les microcapsules comprennent une quantité de PA comprise entre 55 et 95 % en poids, et de préférence entre 60 et 85 % en poids.

Il est important de noter que l'acquisition de cette fonctionnalité de temps de séjour prolongé dans l'intestin grêle ne s'est pas faite au détriment des autres spécifications exigées pour un système galénique du type décrit dans la présente invention. En particulier, le système présente suffisamment de souplesse dans sa composition pour pouvoir être adapté à l'absorption prolongée, pendant des périodes allant par exemple de 5 à 24 heures, d'un grand nombre de PA présentant des solubilité comprises entre quelques milligrammes et quelques centaines de grammes par litre.

Une autre considération à porter au nombre des avantages des microcapsules selon l'invention est que même en cas d'adhérence prolongée à la muqueuse gastro-intestinale, il n'y a pas de risque d'ulcération. En effet, étant donné leur faible granulomètrie, chaque microcapsule ne contient qu'une fraction du PA, représentant typiquement de 1/15 000 à 1/150 000 de la dose administrée, ce qui est très différent du cas des systèmes monolithiques évoqués supra.

Cette faible granulométrie est par ailleurs en soi un facteur permettant une grande régularité de transit ; le transit gastrique étant alors, comme on l'a vu, indépendant de l'ouverture du pylore, et en conséquence, particulièrement rapide.

La présente invention a également pour objet un procédé pour l'obtention notamment des microcapsules selon l'invention telles que définies ci-dessus, ledit procédé consistant essentiellement à :

> a/ sélectionner, ou à défaut fabriquer, des microparticules de PA, de granulométrie comprise entre 50 et 1 000 μm, de préférence entre 100 et 750 μm, et, plus préférentiellement encore, entre 100 et 500 μm,
> b/ préparer une composition d'enrobage par mélange d'un polymère P1, d'un polymère P2, d'un agent plastifiant et d'un agent tensio-actif et/ou lubrifiant dans un système solvant,
> c/ appliquer le mélange obtenu en b/ à la surface des microparticules de PA,
> d/ sécher les microcapsules ainsi obtenues,
> e/ et éventuellement mélanger ces dernières avec au moins un agent antiagglomérant.

Il s'agit là d'une des méthodologies générales intéressantes, qui permet de produire les microcapsules de l'invention d'une manière simple et économique.

La **fig. 1** montre la courbe des concentrations plasmatiques moyennes (chez six sujets) en aténolol (ng/ml) en fonction du temps écoulé en heures (h), après administration de 100 mg d'équivalent aténolol microencapsulé conformément à l'invention (exemple 3).

La **fig. 2** montre la courbe d'absorption cumulée in vivo de l'aténolol (% absorbé), calculée par les méthodes de WAGNER-NELSON et de déconvolution, à partir des concentrations plasmatiques, en fonction du temps écoulé en heures (h) après ingestion des microcapsules (exemple 3).

La **fig. 3** montre la courbe des concentrations plasmatiques moyennes (chez six sujets) en aciclovir (ng/ml) en fonction du temps écoulé en heures (h), après administration de 400 mg d'équivalent aciclovir microencapsulé conformément à l'invention (exemple 4).

La **fig. 4** montre la courbe d'absorption cumulée in vivo de l'aciclovir (% absorbé), calculée par les méthodes de WAGNER-NELSON et de déconvolution, à partir des concentrations plasmatiques, en fonction du temps en heures (h) écoulé après ingestion des microcapsules (exemple 4).

La **fig. 5** montre la courbe des concentrations plasmatiques moyennes (chez six sujets) en captopril (ng/ml) en fonction du temps écoulé en heures (h), après administration de 100 mg d'équivalent captopril microencapsulé conformément à l'invention (exemple 5).

La **fig. 6** montre la courbe d'absorption cumulée in vivo du captopril (% absorbé) calculée par les méthodes de WAGNER-NELSON et de déconvolution, à partir des concentrations plasmatiques, en fonction du temps en heures (h) écoulé après ingestion des microcapsules (exemple 5).

La **fig. 7** montre la courbe des concentrations plasmatiques moyennes (chez six sujets) en cimétidine (ng/ml) en fonction du temps écoulé en heures (h), après administration de 800 mg d'équivalent cimétidine microencapsulé conformément à l'invention (exemple 6).

La **fig. 8** montre montre la courbe d'absorption de la cimétidine (% absorbé) calculée par les méthodes de WAGNER-NELSON et de déconvolution, à partir des concentrations plasmatiques, en fonction du temps en heures (h) écoulé après ingestion des microcapsules (exemple 6).

On conçoit aisément que l'approche descriptive de certains des paramètres essentiels de l'invention, qui sont le temps de séjour des microcapsules de PA dans l'intestin grêle et l'absorption in vivo de ce dernier, soit relativement délicate si on l'aborde de manière directe. Aussi, une alternative envisageable peut être de définir, à la fois le temps le séjour dans l'intestin grêle des microcapsules (administrées per os) et l'absorption in vivo du PA, au travers de la mesure de la concentration plasmatique d'un PA ayant une demi-vie courte dans l'organisme et/ou n'étant pas absorbé au niveau du côlon.

Or, il se trouve qu'à cet égard l'aténolol, la cimétidine et l'aciclovir constituent des traceurs privilégiés, puisqu'ils ne sont pas absorbés au niveau du côlon et que leur durée de demi-vie d'élimination sont, respectivement, de 2 h, 5 h et 1,5 h. Cela sera illustré par les exemples qui suivent.

Ces microcapsules trouvent une partie de leur singularité dans leur structure réservoir que l'homme de l'art distingue bien des systèmes matriciels (cf. Article de C. DUVERNEY et J. P. BENOIT dans "l'Actualité Chimique", décembre 1986 et ouvrage intitulé "Novel drug delivery and its therapeutic application" de L. F. PRESCOTT & W. S. NIMMO, Ed. John WILEY & Sons).

La granulométrie des microcapsules est l'un des paramètres importants de l'invention. Cette granulométrie est déterminée par tamisage. En pratique, cette dernière est, par exemple, comprise entre 100 et 500 microns pour les microcapsules selon l'invention.

La pellicule d'enrobage des microparticules forme, bien entendu, l'une des pièces maîtresses de la présente invention.

Il est du mérite de la demanderesse d'avoir mis au point une composition d'enrobage basée sur la sélection, originale et non arbitraire, de quatre composés dont les fonctionnalités se combinent pour l'obtention des caractéristiques surprenantes visées par l'invention.

L'éthylcellulose et l'acétate de cellulose, susceptibles de constituer P1, sont des polymères solubles dans au moins un solvant organique de température d'ébullition comprise entre 35 et 120 °C.

La polyvinylpyrrolidone et le polyacrylamide formant P2 sont des polymères solubles dans au moins un solvant de P1.

Le plastifiant et l'agent tensio-actifs et/ou le lubrifiant plus particulièrement préférés sont, respectivement, d'une part, l'huile de ricin, le phtalate de diéthyle, le triéthyle citrate et l'acide salicylique, employés seuls ou en mélange entre eux et/ou, d'autre part, le stéarate de magnésium, l'oléate de sodium et/ou le laurate de sorbitan polyoxyéthyléné.

A titre d'exemple de composition d'enrobage plus volontiers mise en oeuvre, on peut citer celle comprenant : éthylcellulose (P1) / polyvinylpyrrolidone (P2) / huile de ricin (plastifiant) / stéarate de magnésium (agent lubrifiant), présents respectivement, dans les teneurs relatives préférées suivantes : 60 - 80 % / 5 - 10 % / 5 - 10 % / 2 - 8 %, les pourcentages étant donnés en poids par rapport au total des composants de l'enrobage.

Cette composition d'enrobage constitue l'une des spécificités originales de la présente invention. Elle est caractérisée par une combinaison intime des quatre composés indiqués ci-dessus. On peut, naturellement, lui adjoindre des adjuvants traditionnellement utilisés dans le domaine du pelliculage, comme des pigments ou des charges.

Pour prévenir les problèmes de mottage des particules enrobées constituant les microcapsules de l'invention, on prévoit, avantageusement, de leur adjoindre au moins un agent anti-agglomérant formé, de préférence, par du talc, de la silice colloïdale ou par un mélange des deux. Cette adjonction se fait, par exemple, selon des quantités de 0,5 à 5 % en poids, de préférence de 1,5 à 3 % en poids.

Les PA utilisés pour la préparation de systèmes à libération contrôlée selon la présente invention peuvent être choisis, à l'exclusion de l'acide acétylsalicylique, parmi au moins l'une des grandes variétés de substances actives, suivantes e.g. : antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti inflammatoires, analgésiques, antiépileptiques, antiparkinsoniens, neuroleptiques, hypnotiques, anxiolytiques, psychostimulants, antimigraineux, antidepresseurs, antitussifs, antihistaminiques ou antiallergiques.

Le PA, dès lors qu'il est médicamenteux, est choisi, de préférence, parmi les composés suivants : pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazépine, ranitidine, énalapril, simvastatine, fluoxétine, alprazolam, famotidine, ganciclovir, famciclovir, spironolactone, 5-asa, quinidine, périndopril, morphine, pentazocine, paracétamol, oméprazole, métoclopramide et leurs mélanges.

Les principes actifs, également concernés par la présente invention, peuvent être choisis parmi les suppléments nutritionnels et/ou diététiques ou leurs mélanges, comme par exemple des vitamines, des acides aminés, des antioxydants ou des oligoéléments ou leurs mélanges.

De façon générale, l'enrobage des particules de PA selon l'invention est obtenu par pulvérisation de la combinaison intime formant la pellicule d'enrobage, en dispersion ou en suspension dans un solvant ou un mélange de solvants organiques.

Le procédé d'enrobage, qui constitue un autre objet de l'invention, s'inscrit dans le cadre des techniques de micro-encapsulation, dont les principales sont résumées dans l'article de C. DUVERNEY et J. P. BENOIT dans "L'actualité chimique", décembre 1986. Plus précisément, la technique considérée est la microencapsulation par pelliculage, conduisant à des systèmes "réservoir" individualisés par opposition aux systèmes matriciels.

De préférence, ce procédé consiste essentiellement à :

a/ sélectionner, ou à défaut fabriquer, des microparticules de PA de granulométrie comprise entre 50 et 1 000 microns, de préférence entre 100 et 750 microns et, plus préférentiellement, entre 100 et 500 microns.

b/ préparer la composition d'enrobage par mélange de P1, de P2, du plastifiant et de l'agent tensio-actif et/ou lubrifiant dans un système solvant,

c/ appliquer le mélange composition d'enrobage/système solvant sur des particules de PA,

d/ sécher les microcapsules ainsi obtenues,

e/ et éventuellement mélanger ces dernières avec au moins un agent anti-agglomérant.

Les solvants convenables pour entrer dans la composition du système solvant sont, par exemple, des cétones, des esters, des solvants chlorés, des alcools de préférence aliphatiques, des alcanes ou des mélanges d'entre eux.

Avantageusement, ces solvants sont des composés en $C_1$-$C_6$, étant précisé que l'acétone, la méthyléthylcétone, le méthanol, l'éthanol, l'isopropanol, le cyclohexane et le chlorure de méthylène sont particulièrement préférés.

Pour aller plus dans le détail de la méthodologie d'enrobage susceptible d'être mise en oeuvre conformément à l'invention, on peut préciser que l'application du mélange composition d'enrobage/système solvant s'opère par pulvérisation sur les particules de PA mises en mouvement, de préférence par agitation mécanique ou par fluidisation.

Pour obtenir des microcapsules selon l'invention, il est nécessaire d'encapsuler des particules de PA de taille comprise entre 50 et 1 000 microns, de préférence entre 100 et 750 microns et, plus préférentiellement, entre 100 et 500 microns.

Les particules de PA de granulométrie désirée et nécessaire à la réalisation des microcapsules selon l'invention peuvent être des cristaux de PA pur et/ou ayant subi un prétraitement par l'une des techniques conventionnelles en la matière, comme par exemple la granulation, en présence d'une faible quantité d'au moins un agent liant classique et/ou d'un agent modifiant les caractéristiques de solubilité intrinsèque du PA.

Selon une modalité intéressante de l'invention, la teneur en PA des particules avant enrobage est comprise entre 75 et 100 % en poids, de préférence entre 95 et 100 % en poids.

La quantité d'enrobant des microcapsules représente de 5 à 40 % du poids des microcapsules enrobées.

La masse spécifique réelle des microcapsules selon l'invention n'est pas critique, mais est préférentiellement comprise entre 1,0 et 1,35 gramme par centimètre cube.

Selon un mode préféré de mise en oeuvre du procédé, conforme à l'invention, de microencapsulation de particules de PA, on prévoit les étapes suivantes :

$a_1$/préparer tout d'abord un mélange comprenant de 70 à 80 % en poids d'un polymère filmogène P1 et 5 à 10 % en poids d'un plastifiant pour 5 à 10 % en poids d'un polymère azoté P2 en solution, soit dans un mélange acétone/alcanol tel que le rapport volumique acétone/alcanol soit compris entre 50/50 et 70/30, soit dans un solvant choisi parmi le cyclohexane, le toluène, le tétrachlorure de carbone, le chloroforme ou le chlorure de méthylène.

$a_2$/mettre en suspension, dans la solution préparée à l'étape précédente, 2 à 8 % en poids d'agent tensio-actif et/ou lubrifiant,,

b/ pulvériser le mélange résultant sur les microparticules de principe actif en lit fluidisé,

c/ sécher les microcapsules à la fin de la pulvérisation en lit fluidisé et/ou à l'étuve,

d/ mélanger les microcapsules ainsi obtenues avec 0,5 à 3 % en poids d'agent anti-adhérent, sur la base de 100 % de produit final obtenu après mélange.

Les microcapsules décrites ci-dessus, et éventuellement obtenues par le procédé également exposé ci-dessus, peuvent être utilisées pour la fabrication de nouvelles préparations pharmaceutiques ou diététiques de divers PA, ayant des performances thérapeutiques ou diététiques optimisées et se présentant de préférence sous forme de comprimés avantageusement délitables, de poudres ou de gélules.

Ces microcapsules sont d'autant plus intéressantes qu'elles sont en outre parfaitement tolérées par l'organisme, notamment au niveau gastrique et par ailleurs obtenables de façon aisée et économique.

La présente invention concerne, en outre, ces nouvelles préparations pharmaceutiques ou diététiques en tant que telles, originales dans leur structure, leur présentation et leur composition. De telles préparations pharmaceutiques ou diététiques sont administrées per os, de préférence par doses journalières uniques.

Il est à noter qu'il peut être intéressant de mélanger dans une même gélule, un même comprimé ou une même poudre, au moins deux types de microcapsules à cinétiques de libération différentes mais comprises dans le cadre caractéristique de l'invention.

On peut également mélanger les microcapsules selon l'invention avec une certaine quantité de PA immédiatement disponible dans l'organisme.

Enfin, un autre objet de l'invention est un système galénique (pharmaceutique ou diététique), de préférence sous forme de comprimé, avantageusement délitable, de poudre ou de gélule, caractérisé en ce qu'il comprend des microcapsules, telles que décrites supra.

L'invention sera mieux expliquée par les exemples ci-après, donnés uniquement à titre d'illustration et permettant de bien comprendre l'invention et de faire ressortir ses variantes de réalisation et/ou de mise en oeuvre, ainsi que ses différents avantages.

## EXEMPLES

### FABRICATION ET EVALUATION PHARMACOCINETIQUE DE MICROCAPSULES SELON L'INVENTION A BASE DE : ATENOLOL, ACYCLOVIR, CAPTOPRIL, CIMETIDINE

### EXEMPLE 1 : PROTOCOLE D'ADMINISTRATION AUX VOLONTAIRES SAINS.

Six sujets de sexe masculin, volontaires considérés comme sains ont été inclus dans chacune des études après avoir satisfait à un examen clinique; ils sont exempts de diabète, maladie cardio-vasculaire, d'affection hépatique ou rénale et de problèmes gastro-intestinaux. Chaque sujet a subi en outre un examen hématologique et biochimique.

Les sujets n'ont pris aucun traitement médicamenteux, y compris des antiacides et des analgésiques pendant une période d'au moins 1 semaine avant le début de l'étude. Ils sont à jeun de nourriture et/ou de boissons depuis la veille (22 h) et le resteront pendant 2 à 4 heures après administration des traitements.

Les gélules de la formulation micro-encapsulée à tester ont été fabriquées selon les Bonnes Pratiques de Fabrication et les Bonnes Pratiques Cliniques.

Les comprimés de la forme de référence ont été achetés en pharmacie.

Chaque sujet qui reçoit les deux formulations par voie orale, au cours des deux périodes séparées chacune par 7 à 15 jours d'une étude en essai croisé randomisé, est son propre témoin. Les gélules et les comprimés sont avalés avec un verre d'eau.

Au cours des 24 premières heures, les prélèvements sanguins (8 ml) sont effectués au moyen d'un cathéter en téflon à ailettes, qui est rincé à intervalles réguliers avec une solution d'héparine diluée au 1/10$^{ème}$ dans du sérum physiologique.

Les autres prélèvements sont effectués par prélèvement direct au pli du coude, dans les heures qui suivent les 24 premières heures ou lorsque le cathéter n'est plus supporté par le volontaire.

Les prélèvements sanguins sont réalisés quelques instants avant l'administration du médicament (15 minutes environ) et à 0,5 - 1 - 2 - 3 - 4 - 6 - 8 - 10 - 12 - 16 - 24 - 36 - 48 - 72 et 96 heures après la prise.

Chaque échantillon de sang a été immédiatement centrifugé à 4 °C et le plasma recueilli dans des tubes qui sont stockés à -20 °C à l'abri de la lumière jusqu'à analyse du principe actif.

Tous les échantillons plasmatiques sont analysés pour obtenir la concentration du produit inchangé en fonction du temps.

## EXEMPLE 2 : ANALYSE DE DISSOLUTION ET DE L'ABSORPTION IN VIVO CHEZ L'HOMME.

L'évaluation de la dissolution et de l'absorption in vivo chez l'homme a été réalisée en utilisant les techniques indirectes faisant appel au traitement mathématique des concentrations plasmatiques réellement mesurées en fonction du temps; ces techniques sont connues sous le nom d'analyses de WAGNER-NELSON et de LOO RIEGELMAN ou encore analyses par des techniques de déconvolution (Umesh V. Banakar, Chetan D. Lathia and John H. Wood (1992). Interpretation of dissolution rate data and techniques of in vivo dissolution. In "Pharmaceutical Dissolution Testing ", Marcel Dekker, Inc. New York, pp 189-249).

Les techniques de WAGNER-NELSON ou de LOO-RIEGELMAN (WAGNER-NELSON est utilisé pour un modèle ouvert à un compartiment et les techniques de LOO-RIEGELMAN sont utilisées pour un modèle ouvert à deux compartiments) utilisent la dérivée de l'équation des concentrations plasmatiques en fonction du temps pour déterminer la fonction d'absorption.

L'analyse par déconvolution numérique permet par ailleurs d'obtenir non seulement la constante de vitesse de libération à partir de la forme pharmaceutique, mais également le profil de libération du médicament dans le tractus gastro-intestinal à partir d'une forme solide.

L'intérêt de l'utilisation de la technique de déconvolution est qu'elle permet à la fois de décrire la fraction du médicament atteignant la circulation systémique, mais également le temps de dissolution in vivo de ce médicament.

Les concentrations plasmatiques ont été analysées en utilisant le logiciel SIPHAR fonctionnant sur un micro-ordinateur compatible IBM. Chacun des profils plasmatiques après la prise du médicament ont été mathématiquement ajustés en utilisant un algorithme itératif selon la fonction des moindres carrés. Pour les profils d'absorption par voie orale, une fonction mathématique de premier ordre a été utilisé ; un modèle mathématique avec une ou deux exponentielles décrit la décroissance des taux plasmatiques dans l'organisme (équation 1). Un facteur de pondération a été utilisé si besoin.

$$C_p = \sum_{i=1}^{n} C_i e^{-\lambda_i t} \qquad\qquad 1$$

$C_p$ représente la concentration plasmatique à l'instant t, $C_i$ est le coefficient et $\lambda_i$ le facteur de l'exponentielle pour le n$^{ième}$ terme. Une équation exponentielle négative a été utilisée pour décrire la phase d'absorption.

Les taux plasmatiques maximum ($C_{max}$) et le temps nécessaire pour atteindre ce taux plasmatique ($T_{max}$) ont été obtenus à partir des valeurs réellement mesurées. Les surfaces sous courbes (AUC), déterminées soit jusqu'au dernier point mesuré ou extrapolées à l'infini, ont été calculées soit par la méthode des trapèzes (YEH and KWAN, 1978) soit par intégration directe de l'équation exponentielle. La demi-vie ($t_{1/2}$) de chacune des phases de décroissance du médicament dans l'organisme a été obtenue à partir de l'équation 2 en utilisant respectivement les constantes de l'exponentielle obtenues à partir de la courbe plasmatique.

$$t_{1/2} = \frac{0.693}{\lambda i} \qquad\qquad 2$$

Les paramètres obtenus sont automatiquement intégrés dans une base de données de l'ordinateur comportant le code de l'étude ; cette base de données peut être automatiquement appelée soit pour obtenir des tableaux, soit pour réaliser l'analyse statistique.

Les données obtenues in vivo ont été analysées ensuite en utilisant le même programme SIPHAR. Les données plasmatiques ainsi analysées ont été utilisées pour déterminer le profil d'absorption de la fraction médicamenteuse absorbée, à partir des équations de WAGNER-NELSON, LOO-RIEGELMAN ou des analyses de déconvolution déjà décrites, selon la technique de HILL ou WEIBULL et l'algorithme de POWELL.

## EXEMPLE 3 : PROFILS D'ABSORPTION D'UNE FORMULATION MICRO-ENCAPSULEE D'ATENOLOL.

*3.1 ENCAPSULATION DE L'ATENOLOL CONFORMEMENT A L'INVENTION :*

Le principe actif se présente sous forme d'une poudre de microcristaux. La granulométrie de cette poudre est mesurée grâce à un granulomètre laser Coulter LS 130 dans l'heptane. On obtient les diamètres suivants :

-   Dmoyen = 6,8 microns (D moyen en volume),
-   80 % de la masse comprise entre 1,1 et 14,7 $\mu$m. 2 901 g de poudre d'aténolol sont ensuite mélangés dans un granulateur Lodige M5 GRI à 86,8 g de PVP et à 1 953,5 g d'eau purifiée. Les microparticules sont tamisées entre 200 et 315 microns et l'on en récupère ainsi 925 g.

On vaporise 199,6 g d'eau purifiée sur ces microparticules.
299,4 g de ces microparticules sont pelliculés par spray coating dans un appareil Uniglatt par la composition suivante :

| | |
|---|---|
| - Ethylcellulose | 44, 7 g |
| - PVP | 4,8 g |
| - Huile de ricin | 4,8 g |
| - Stéarate de magnésium | 6,1 g |
| - Acétone | 479,0 g |
| - Isopropanol | 53,0 g |
| - Acide salicylique | 15, 1 g |

Après tamisage entre 200 et 315 microns, on obtient 202,8 g de microcapsules dont la granulométrie est la suivante :

-   Dmoyen = 272 microns (D moyen en volume),
-   80 % de la masse correspond à des microcapsules de diamètre compris entre 198 et 355 microns.

La teneur en principe actif du produit pelliculé est de 74 %.

*3.2. MESURE DE L'ABSORPTION IN VIVO CHEZ DES VOLONTAIRES SAINS APRES ADMINISTRATION DE MICROCAPSULES D'ATENOLOL :*

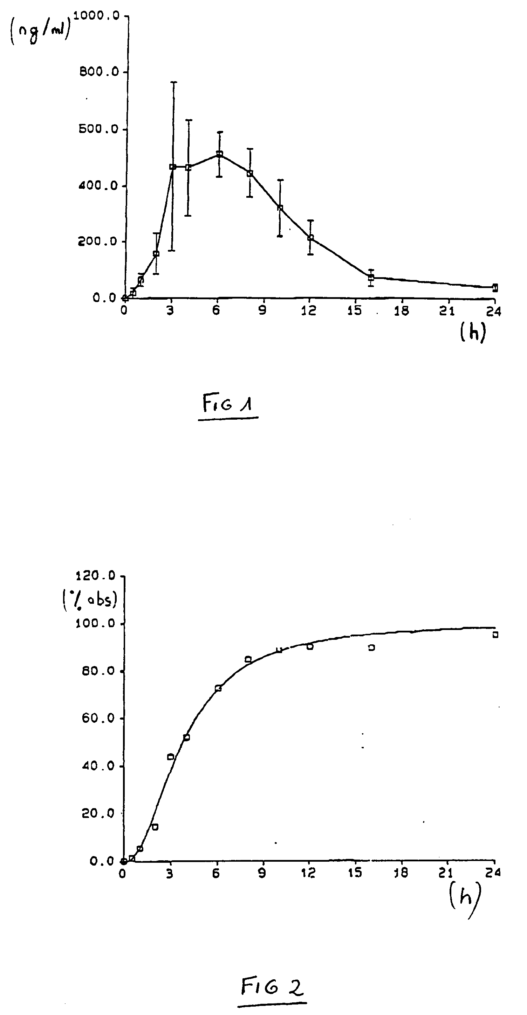

Après administration d'une dose de 100 mg d'aténolol encapsulé, à des volontaires sains, au cours d'une étude randomisée croisée, comparativement au produit de référence Tenormin (100 mg) suivi de prélèvements d'échantillons de sang tels qu'indiqués dans l'exemple 1, les échantillons plasmatiques ont été analysés pour déterminer l'aténolol inchangé. Préalablement à l'extraction, une solution d'étalon interne (1 ml) a été ajoutée à chacun des échantillons plasmatiques ; l'étalon interne et le principe actif ont été ensuite extraits du plasma par une extraction solide-liquide.
L'analyse a été réalisée par chromatographie liquide haute performance en utilisant un chromatographe HP 1050. La séparation chromatographique a été faite sur un colonne (15 cm x 4 mm) comportant une phase superspher 100RP18 de 4 $\mu$m, et en utilisant une phase mobile d'alcool méthylique/tampon phosphate (20/80 ; v/v) ; le débit de la phase mobile est de 0,6 ml/min ; la détection du principe actif a été réalisée par fluorimétrie.
Dans ces conditions, l'aténolol a pu être mesuré avec un temps de rétention de 30 min. La droite d'étalonnage est linéaire sur les concentrations de 2,5-500 ng.ml$^{-1}$.
La limite de détection du produit est de 2.5 ng.ml$^{-1}$.
Les taux plasmatiques moyens de l'aténolol ($\pm$ Déviations Standard SD) sont présentés dans le tableau 1 ci-après et dans la **fig. 1** annexée :

TABLEAU 1

| Heures | Concentrations en ng/ml |
|---|---|
| 0 | 0,00 $\pm$ 0,00 |
| 0,5 | 17,60 $\pm$ 14,01 |
| 1 | 89,07 $\pm$ 56,05 |
| 2 | 202,41 $\pm$ 88,71 |
| 3 | 488,02 $\pm$ 241,50 |
| 4 | 458,47 $\pm$ 133,51 |
| 6 | 296,52 $\pm$ 61,83 |
| 8 | 202,44 $\pm$ 45,95 |
| 10 | 146,03 $\pm$ 23,70 |
| 12 | 120,46 $\pm$ 20,57 |
| 16 | 74,03 $\pm$ 12,48 |
| 24 | 38,42 $\pm$ 6,87 |
| 36 | 14,32 $\pm$ 2,89 |
| 48 | 6,95 $\pm$ 2,35 |
| 72 | 0,88 $\pm$ 2,14 |

## TABLEAU 1 (suite)

| Heures | Concentrations en ng/ml |
|---|---|
| 96 | $0,00 \pm 0,00$ |

A partir de ces taux plasmatiques, l'analyse de l'absorption a été réalisée en utilisant la méthode de WAGNER-NELSON ou la méthode de déconvolution. L'une et l'autre de ces techniques permettent de déterminer les temps nécessaires pour atteindre 10 - 50 et 90 % du médicament absorbé ainsi que le temps d'absorption moyen ($T_D$) et la constante $\gamma$ de l'équation de WEIBULL.
L'une et l'autre de ces techniques ont été utilisées pour éviter le biais introduit par le fait que la substance de référence est un produit commercial sous forme comprimé et non pas une forme d'administration par voie intraveineuse. La **fig. 2** annexée montre le pourcentage absorbé in vivo en fonction du temps.
Les paramètres obtenus sont présentés dans le tableau 2 ci-dessous :

## TABLEAU 2

| Paramètres | WAGNER- NELSON |
|---|---|
| $T_{10\%}$ | 1,50 heures |
| $T_{50\%}$ | 3,77 heures |
| $T_{90\%}$ | 17,01 heures |
| $T_D$ | 5,40 heures |
| $\gamma$ | 0,83 heures |

La **fig. 2** et le tableau 2 montrent que l'absorption in vivo se prolonge au-delà du temps de transit dans l'intestin grêle usuellement admis, qui est de $3 \pm 1$ h.
Ainsi, la forme galénique selon l'invention (microcapsules) présente un temps de transit supérieur au temps de transit physiologique, l'aténolol n'étant pas absorbé au niveau du côlon.

## EXEMPLE 4 : PROFIL D'ABSORPTION D'UNE FORMULATION MICRO-ENCAPSULEE D'ACICLOVIR.

### 4.1. *ENCAPSULATION DE L'ACICLOVIR CONFORMEMENT A L'INVENTION:*

Le principe actif se présente sous forme d'une poudre de microcristaux. La granulométrie de cette poudre est mesurée grâce à un granulomètre laser Coulter LS 130 dans l'heptane. On obtient les diamètres suivants :

- Dmoyen = 8,6 microns (D moyen en volume),

- diamètre maximal pour 95 % de la masse : 28 microns.

1 500 g de poudre d'aciclovir sont ensuite mélangés dans un granulateur Bouvard Erweka à 45 g de PVP et à 409 g d'une solution eau / isopropanol 50/50 m/m. Les microparticules sont tamisées entre 315 et 500 microns et l'on en récupère ainsi 973 g. 350 g de ces microparticules sont pelliculés par spray coating dans un appareil Uniglatt par la composition suivante :

| - Ethylcellulose | 15,5 g |
|---|---|
| - PVP | 1,7 g |
| - Huile de ricin | 1,7 g |
| - Stéarate de magnésium | 2,1 g |
| - Acétone | 166,0 g |
| - Isopropanol | 18,0 g |

On obtient des microcapsules dont les caractéristiques granulométriques sont les suivantes :

- Dmoyen = 393 $\mu$m (D moyen en volume),
- 80 % de la masse correspond à des microcapsules de diamètre compris entre 253 et 561 microns.

La teneur en principe actif du produit pelliculé est de 88 %.

### 4.2. *MESURE DE L 'ABSORPTION IN VIVO CHEZ DES VOLONTAIRES SAINS APRES ADMINISTRATION DE MICROCAPSULES D'ACICLOVIR :*

Après administration de 400 mg d'aciclovir micro-encapsulé, à des volontaires sains au cours d'une étude croisée, randomisée, comparativement au Zovirax (2 x 200 mg) comme produit de référence, des échantillons de sang ont été prélevés ainsi que cela a été décrit dans l'exemple 1 ; 1 ml de plasma ainsi obtenu a été mélangé avec 300 $\mu$l de $HClO_4$ 3M et agité pendant 15 sec. sur un Vortex. Les échantillons sont alors centrifugés pendant 5 min. et le surnageant prélevé et introduit dans des flacons pour échantillonneur automatique. 50 $\mu$l sont alors injectés dans un système HPLC. La séparation chromatographique a été réalisée grâce à une colonne en phase inverse $C_{18}$ utilisant une phase mobile composée d'acétonitrile et de $HClO_4$ 0,02 M en observant le gradient suivant: 100 % $HClO_4$ pendant 5,9 min, puis 20 % HClO4 et 80 % acétonitrile de la 6ème à la 14ème minute, enfin 100 % d'HClO4 jusqu'à la fin de l'analyse. La détection a été réalisée grâce à un fluorimètre (longueur d'onde d'excitation : 260 nm, longueur d'onde d'émission : 375 nm).

La linéarité a été mesurée de 10 à 2 000 ng/ml et la limite de quantification obtenue est de 10 ng/ml.

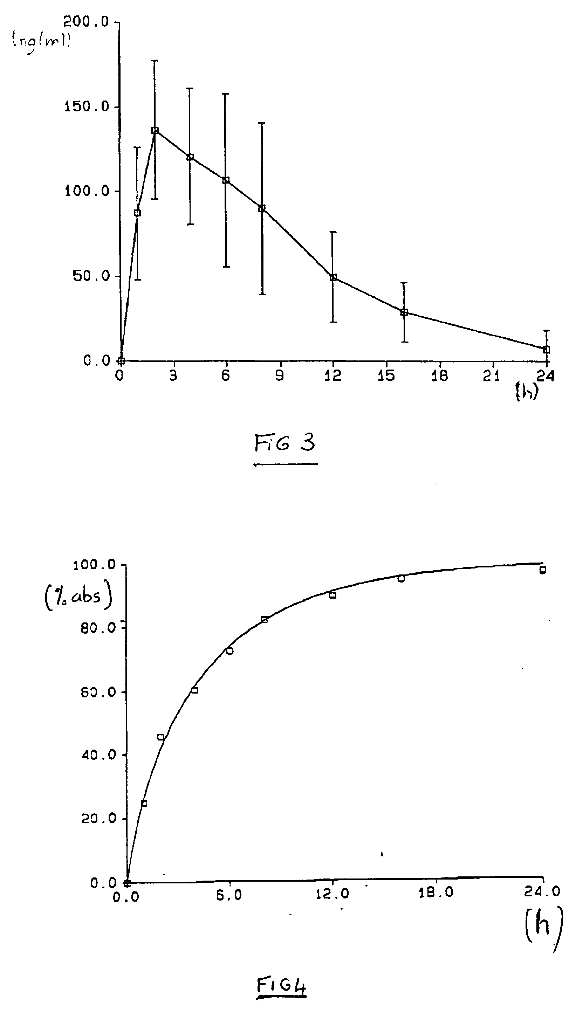

La validation de la méthode a été réalisée en observant les règles de Bonnes Pratiques de Laboratoire (BPL). Les taux plasmatiques (± SD) d'aciclovir sont présentés dans le tableau 3 suivant et dans la **fig. 3** annexée :

TABLEAU 3

| Heures | Concentrations en ng/ml |
|--------|-------------------------|
| 0      | 0,00 ± 0,00             |
| 0,5    | 50,00 ± 0,00            |
| 1      | 103,83 ± 39,44          |
| 2      | 128,03 ± 33,88          |
| 4      | 104,03 ± 13,08          |
| 6      | 56,3 ± 13,89            |
| 8      | 26,60 ± 21,14           |
| 12     | 4,08 ± 10,00            |
| 16     | 3,65 ± 8,94             |
| 24     | 3,78 ± 9,27             |

A partir de ces taux plasmatiques, l'analyse de l'absorption a été réalisée en utilisant la méthode de WAGNER-NELSON ou la méthode de déconvolution. L'une et l'autre de ces techniques permettent de déterminer les temps nécessaires pour atteindre 10 - 50 et 90 % du médicament absorbé ainsi que le temps d'absorption moyen ($T_D$) et la constante $\gamma$ de l'équation de WEIBULL.

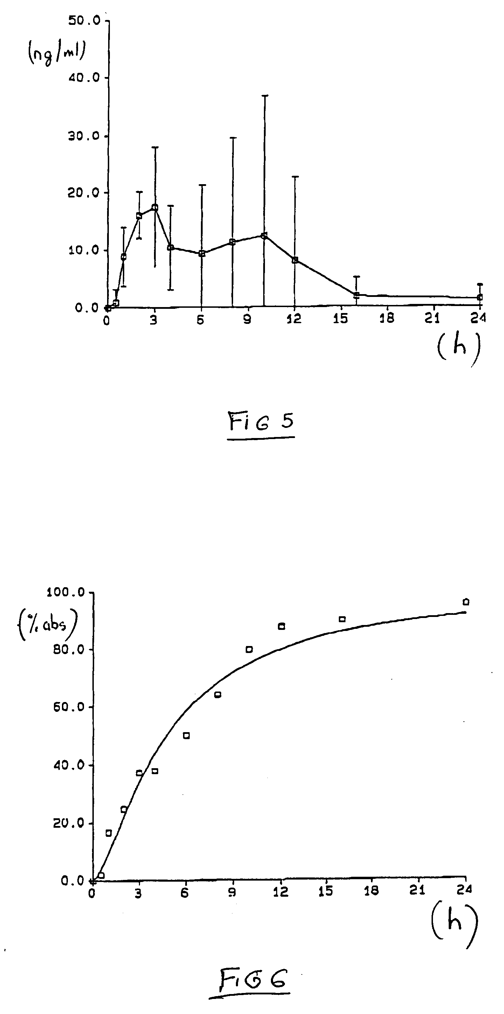

L'une et l'autre de ces techniques ont été utilisées pour éviter le biais introduit par le fait que la substance de référence est un produit commercial sous forme comprimé et non pas une forme d'administration par voie intraveineuse. La **fig. 4** annexée montre le pourcentage absorbé in vivo en fonction du temps.

Les paramètres obtenus sont présentés dans le tableau 4 ci-dessous :

TABLEAU 4

| Parmè- tres | Déconvolu- tion |
|-------------|-----------------|
| $T_{10\%}$  | 0, heures       |
| $T_{50\%}$  | 2,60 heures     |
| $T_{90\%}$  | 12,58 heures    |
| $T_D$       | 4,04 heures     |
| $\gamma$    | 0,82 heures     |

La **fig. 4** et le tableau 4 montrent que l'absorption in vivo se prolonge au-delà du temps de transit dans l'intestin grêle usuellement admis, qui est de 3 ± 1 h.

Ainsi, la forme galénique selon l'invention (microcapsules) présente un temps de transit supérieur au temps de transit physiologique.

**EXEMPLE 5 : PROFIL D'ABSORPTION D'UNE FORMULATION MICRO-ENCAPSULEE DE CAPTOPRIL.**

5.1 *ENCAPSULATION DU CAPTOPRIL CONFORMEMENT A L'INVENTION :*

Le principe actif se présente sous forme d'une poudre de microcristaux. La granulométrie de cette poudre est mesurée grâce à un granulomètre laser Coulter LS 130 dans l'heptane. On obtient les diamètres suivants :

-    Dmoyen = 18 microns (D moyen en volume),
-    diamètre maximal pour 95 % de la masse : 52,7 microns.

2 800,6 g de poudre d'aciclovir sont ensuite mélangés dans un granulateur Lodige M5 GRI à 87,1 g de PVP et à 1 301 g d'eau purifiée. Les microparticules sont tamisées entre 200 et 315 microns et l'on en récupère ainsi 973 g.

On vaporise 200 g d'eau purifiée sur ces microparticules.

300 g de ces microparticules sont pelliculés par spray coating dans un appareil Uniglatt par la composition suivante :

| | |
|-----------------------|-------------|
| - Ethylcellulose      | 120,30 g    |
| - PVP                 | 13,00 g     |
| - Huile de ricin      | 13,00 g     |
| - Stéarate de magnésium | 16,26 g   |
| - Acétone             | 1 284,70 g  |
| - Isopropanol         | 142,70 g    |

Après tamisage entre 200 et 315 microns, on obtient 57 g de microcapsules dont la granulométrie est la suivante :

-    Dmoyen = 332 $\mu$m (D moyen en volume),
-    Intervalle de diamètre pour 80 % de la masse : 254 et 421 microns.

La teneur en principe actif du produit pelliculé est de 56,2 %.

## 5.2. *MESURE DE L'ABSORPTION IN VIVO CHEZ DES VOLONTAIRES SAINS APRES ADMINISTRATION DE MICROCAPSULES DE CAPTOPRIL :*

Le captopril micro-encapsulé a été administré chez des volontaires sains (100 mg) au cours d'un essai croisé, randomisé en prenant comme référence le Lopril 100 mg. Les échantillons de sang prélevés selon l'exemple 1 ont permis d'obtenir des échantillons de plasma (100 μl) dans lesquels ont été rajoutés 50 ng de S-benzyl captopril utilisé comme étalon interne; le mélange ainsi obtenu est placé dans des tubes à vis avec 900 μl d'eau et 1 ml d'acide chlorhydrique 0,5 N. Après agitation sur un Vortex, 5 ml de dichlorométhane ont été ajoutés à chacun des tubes. La procédure d'extraction a été réalisée pendant une quinzaine de minutes par agitation vigoureuse. Les tubes sont alors centrifugés à 3 500 rpm pendant 5 min. et la phase organique transférée dans un tube de 10 ml en verre et évaporée à sec sous un flux d'azote à 45 °C. Le résidu ainsi obtenu est reconstitué avec 1 ml d'une solution tampon à pH=3 et le mélange introduit dans une cartouche d'élution composée d'une phase inverse $C_{18}$ préalablement conditionnée avec de l'eau et du méthanol. Après un lavage à l'eau, la colonne est éluée avec 1 ml de méthanol et l'éluat évaporé sous un flux d'air d'azote. 50 μl d'une solution d'éthanol saturée en carbonate de potassium et 50 μl d'une solution de bromure de pentafluorobenzyl à 1 % dans l'acétonitrile sont rajoutés au résidu. Après 1,5 h de dérivatisation à 80 °C, le mélange est évaporé. Sur le résidu sont ajoutés 500 μl d'acétonitrile et 2 μl du mélange ainsi obtenu sont injectés dans un chromatographe en phase gazeuse couplé à un spectromètre de masse. La séparation chromatographique a été réalisée sur une colonne capillaire de silice fondue (8 m x 0,25 mm) traitée avec une phase apolaire stationnaire OV 1701. Les conditions chromatographiques étaient les suivantes :

- température initiale du four : 160 °C,
- gradient : 15 °C/minute,
- température finale du four : 280 °C,
- température du segment : 280 °C.

L'analyse spectrométrique a été réalisée en mode ionisation chimique négative en utilisant du méthane N45 comme gaz réactif et une pression à la source de 0,5 Torr. La validation de la méthode a été réalisée en suivant les Bonnes Pratiques de Laboratoire (BPL). Les taux plasmatiques (± SD) du captopril sont présentés dans le tableau 5 suivant et dans la **fig. 5** annexée :

TABLEAU 5

| Heures | Concentrations en ng/ml |
|--------|-------------------------|
| 0      | 0,00 ± 0,00             |
| 0,5    | 0,93 ± 2,27             |
| 1      | 8,85 ± 5,15             |
| 2      | 16,05 ± 4,03            |
| 3      | 17,49 ± 10,51           |
| 4      | 10,40 ± 7,42            |
| 6      | 9,26 ± 12,03            |
| 8      | 11,25 ± 18,28           |
| 10     | 12,33 ± 24,50           |
| 12     | 8,00 ± 14,57            |
| 16     | 1,78 ± 3,21             |
| 24     | 1,31 ± 2,32             |
| 36     | 0,00 ± 0,00             |
| 48     | 0,00 ± 0,00             |
| 72     | 0,00 ± 0,00             |
| 96     | 0,00 ± 0,00             |

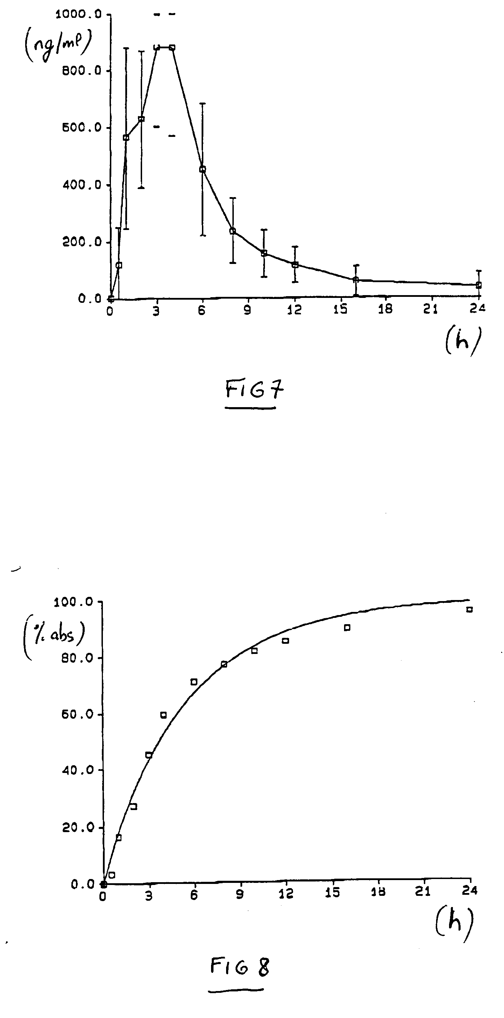

A partir de ces taux plasmatiques, l'analyse de l'absorption a été réalisée en utilisant la méthode de WAGNER-NELSON ou la méthode de déconvolution. L'une et l'autre de ces techniques permettent de déterminer les temps nécessaires pour atteindre 10 - 50 et 90 % du médicament absorbé ainsi que le temps d'absorption moyen ($T_D$) et la constante γ de l'équation de WEIBULL. L'une et l'autre de ces techniques ont été utilisées pour éviter le biais introduit par le fait que la substance de référence est un produit commercial sous forme comprimé et non pas une forme d'administration par voie intraveineuse. La **fig. 6** annexée montre le pourcentage absorbé in vivo en fonction du temps. Les résultats obtenus sont présentés dans le tableau 6 ci-dessous :

TABLEAU 6

| Paramètres | Déconvolution (heures) |
|------------|------------------------|
| $T_{10\%}$ | 0,78                   |
| $T_{50\%}$ | 5,99                   |
| $T_{90\%}$ | 16,54                  |
| $T_D$      | 7,22                   |

## TABLEAU 6 (suite)

| Paramètres | Déconvolution (heures) |
|---|---|
| $\gamma$ | 1,04 |

La **fig. 6** et le tableau 6 montrent que l'absorption in vivo se prolonge au-delà du temps de transit dans l'intestin grêle usuellement admis, qui est de $3 \pm 1$ h.

Ainsi, la forme galénique selon l'invention (microcapsules) présente un temps de transit supéreiur au temps de transit physiologique.

## EXEMPLE 6 : PROFIL D'ABSORPTION D'UNE FORMULATION MICRO-ENCAPSULEE DE CIMETIDINE.

### 6.1. *ENCAPSULATION DE LA CIMETIDINE CONFORMEMENT A L'INVENTION :*

Le principe actif se présente sous forme d'une poudre de microcristaux. La granulométrie de cette poudre est mesurée grâce à un granulomètre laser Coulter LS 130 dans l'heptane. On obtient les diamètres suivants :

- Dmoyen = 19,8 microns (D moyen en volume)
- intervalle des diamètres pour 80 % de la masse : 1,8 et 41,4 microns.

2 899,8 g de poudre de cimétidine sont ensuite mélangés dans un granulateur Lödige M5 GRI à 88,9 g de PVP et 1 039,2 g d'eau purifiée. Les microparticules sont tamisées entre 200 et 315 microns et l'on en récupère ainsi 907 g.

On vaporise 198,3 g d'eau purifiée sur ces microparticules.

299,8 g de ces microparticules sont pelliculés par spray coating dans un appareil Uniglatt par la composition suivante :

| | |
|---|---|
| - Ethylcellulose | 54,40 g |
| - PVP | 5,90 g |
| - Huile de ricin | 5,90 g |
| - Stéarate de magnésium | 7,37 g |
| - Acétone | 580,00 g |
| - Isopropanol | 64,40 g |
| - Acide salicylique | 18,40 g |

Après tamisage entre 200 et 315 microns, on obtient 60 g de microcapsules dont la granulométrie est la suivante :

- Dmoyen = 308 microns (D moyen en volume),
- 80 % de la masse correspond à des microcapsules de diamètre compris entre 219,4 et 413,4 microns.

La teneur en principe actif du produit pelliculé est de 64 %.

### 6.2. *MESURE DE L'ABSORPTION IN VIVO CHEZ DES VOLONTAIRES SAINS APRES ADMINISTRATION DE MICROCAPSULES DE CIMETIDINE :*

La formulation micro-encapsulée de cimétidine (800 mg) a été administrée à des volontaires sains, au cours d'une étude réalisée selon un essai croisé randomisé, par rapport à un produit de référence le Tagamet effervescent en solution (800 mg). Des échantillons de sang ont été prélevés ainsi que cela a été décrit dans l'exemple 1 ; et 0,5 ml des échantillons plasmatiques ainsi obtenus ont été purifiés à travers une colonne d'extraction $C_{18}$ Bond Elut conditionnée par 1 ml de méthanol et 1 ml d'eau ultra-pure. Après lavage par 1 ml d'eau l'échantillon plasmatique a été élué avec 1 ml de la phase mobile utilisée en HPLC; 10 $\mu$l d'une solution d'étalon interne (200 $\mu$g/ml de procaïnamide dans l'eau) ont été alors ajoutés à l'échantillon et 50 $\mu$l ont été injectés dans un système de chromatographie haute performance.

La séparation chromatographique a été réalisée sur une colonne Macherey Nagel Sphérisorb 80-3 CN 3$\mu$m (125 x 8 x4 mm) en utilisant une phase mobile composée d'acétonitrile et d'un tampon phosphate pH = 5 5 mmole (50/50, v/v). La détection a été réalisée en spectrophotométrie U.V.

Des droites d'étalonnage ont été préparées par ajout d'un volume constant de solution contenant des quantités croissantes de cimétidine. Ensuite les échantillons ont été traités de la même manière que les échantillons plasmatiques prélevés sur les volontaires sains.

Les concentrations ainsi obtenues ont été de : 0,05 - 0,1 - 0,2 - 0,5 - 1 - 2 - 5 - 10 $\mu$g/ml.

Les concentrations de cimétidine dans les échantillons plasmatiques provenant des volontaires sains ont été calculés en utilisant une droite d'étalonnage renouvelée chaque jour. Les concentrations ont été obtenues en mesurant le rapport des hauteurs des pics chromatographiques de la cimétidine et de son étalon interne.

La courbe de calibration n'a pas été forcée par 0 et un facteur de pondération de $1/C^2$ a été utilisé. La limite de détection a été estimée à 0,025 $\mu$g/ml et la limite de quantification à 0,05 $\mu$g/ml.

Les taux plasmatiques moyens ($\pm$ SD) sont présentés dans le tableau 7 suivant et la **fig. 7** annexée.

TABLEAU 7

| Heures | Concentrations en ng/ml |
|--------|-------------------------|
| 0,0 | 0,00 ± 0,00 |
| 0,5 | 119,83 ± 133,01 |
| 1 | 565,00 ± 317,51 |
| 2 | 630,17 ± 239,82 |
| 3 | 884,17 ± 281,23 |
| 4 | 882,67 ± 311,78 |
| 6 | 453,67 ± 230,47 |
| 8 | 237,67 ± 115,01 |
| 10 | 156,83 ± 83,55 |
| 12 | 117,17 ± 63,57 |
| 16 | 59,17 ± 53,82 |
| 24 | 39,17 ± 50,55 |
| 36 | 10,33 ± 25,31 |
| 48 | 0,00 ± 0,00 |
| 72 | 0,00 ± 0,00 |
| 96 | 0,00 ± 0,00 |

A partir de ces taux plasmatiques, l'analyse de l'absorption a été réalisée en utilisant la méthode de WAGNER-NELSON ou la méthode de déconvolution. L'une et l'autre de ces techniques permettent de déterminer les temps nécessaires pour atteindre 10 - 50 et 90 % du médicament absorbé ainsi que le temps d'absorption moyen ($T_D$) et la constante $\gamma$ de l'équation de WEIBULL.

L'une et l'autre de ces techniques ont été utilisées pour éviter le biais introduit par le fait que la substance de référence est un produit commercial sous forme comprimé et non pas une forme d'administration par voie intraveineuse. La **fig. 8** montre le pourcentage absorbé in vivo en fonction du temps.

Les résultats obtenus sont présentés dans le tableau 8 ci-dessous.

TABLEAU 8

| Paramètres | Déconvolution (heures) |
|-------------|------------------------|
| $T_{10\%}$ | 0,76 |
| $T_{50\%}$ | 3,33 |
| $T_{90\%}$ | 16,94 |
| TD | 5,39 |

TABLEAU 8 (suite)

| Paramè-tres | Déconvolution (heures) |
|-------------|------------------------|
| $\gamma$ | 0,96 |

La **fig. 8** et le tableau 8 montrent que l'absorption in vivo se prolonge au-delà du temps de transit dans l'intestin grêle usuellement admis, qui est de 3 ± 1 h.

Ainsi, la forme galénique selon l'invention (microcapsules) présente un temps de transit supérieur au temps de transit physiologique, la cimétidine n'étant pas absorbée au niveau du côlon.

**Revendications**

1. Microcapsules, de type réservoir, contenant au moins un Principe Actif médicamenteux et/ou nutritionnel (PA), à l'exclusion de l'acide acétylsalicylique (ASA), destinées à l'administration par voie orale, caractérisées :

   - en ce qu'elles sont constituées par des particules de PA recouvertes chacune d'une pellicule d'enrobage de composition suivante :

     1 - au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, l'éthylcellulose et/ou l'acétate de cellulose étant particulièrement préférés;

     2 - au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N-vinylamide et/ou un poly-N-vinyl-lactame, le polyacrylamide et/ou la polyvinylpyrrolidone étant particulièrement préférés;

     3 - au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin, l'acide salicylique et la cutine, l'huile de ricin étant particulèrement préférée ;

     4 - au moins un agent tensio-actif et/ou lubrifiant, présent à raison de 2 à 20, de préférence de 4 à 15 % en poids sur sec par rapport à la masse totale de la composition d'enrobage et choisi parmi les tensio-actifs anioniques, de préférence les sels alcalins ou alcalinoterreux des acides gras, l'acide stéarique et/ou oléique étant préférés, et/ou parmi les tensio-actifs non ioniques, de préférence les esters de sorbitan polyoxyéthylénés et/ou les dérivés de l'huile de ricin polyoxyéthylénés, et/ou parmi les agents lubrifiants comme les stéarates, de préférence de calcium, de magnésium, d'aluminium ou de zinc, ou comme le stéarylfumarate, de préférence de sodium, et/ou le béhénate de glycérol ; ledit agent pouvant comprendre un seul ou un mélange des susdits produits ;

- en ce qu'elles possèdent une granulométrie comprise entre 50 et 1 000 microns, de préférence entre 100 et 750 microns, et, plus préférentiellement encore, entre 100 et 500 microns,
- en ce qu'elles sont conçues de manière à pouvoir séjourner dans l'intestin grêle pendant un temps d'au moins 5 heures environ, de préférence au moins 7 heures environ et, plus préférentiellement encore, pendant un temps compris entre environ 8 heures et environ 24 heures et permettre ainsi l'absorption du PA pendant au moins une partie de leur temps de séjour dans l'intestin grêle.

2. Microcapsules selon la revendication 1, caractérisées en ce qu'elles comprennent une quantité de PA comprise entre 55 et 95 % en poids, et de préférence entre 60 et 85 % en poids.

3. Microcapsules selon la revendication 1 ou 2, caractérisées en ce que la composition de la pellicule d'enrobage comprend de 60 à 80 % d'éthylcellulose, de 5 à 10 % de polyvinylpyrrolidone, de 5 à 10 % d'huile de ricin et 2 à 8 % de stéarate de magnésium.

4. Microcapsules selon l'une quelconque des revendications 1 à 3, caractérisées en ce qu'elles sont mélangées avec 0,5 à 5 % en poids, de préférence 1,5 à 3 % en poids, d'au moins un agent anti-agglomérant formé, de préférence, par du talc, de la silice colloïdale ou par un mélange des deux.

5. Microcapsules selon l'une quelconque des revendications 1 à 4, caractérisées en ce que le PA mis en oeuvre appartiennent à au moins l'une des familles de substances actives suivantes : antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques.

6. Microcapsules selon la revendication 5, caractérisées en ce que le PA est choisi parmi les composés suivants : pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazepine, ranitidine, énalapril, simvastatine, fluoxétine, alprazolam, famotidine, ganciclovir, famciclovir, spironolactone, 5-asa, quinidine, périndopril, morphine, pentazocine, paracétamol, oméprazole, métoclopramide et leurs mélanges.

7. Microcapsules selon l'une quelconque des revendications 1 à 6, caractérisées en ce que le PA est constitué par au moins un supplément nutritionnel et/ou diététique, de préférence choisi parmi les vitamines, les acides aminés, les oligoéléments, les antioxydants et leurs mélanges.

8. Procédé pour l'obtention des microcapsules selon l'une quelconque des revendications 1 à 7, caractérisé en ce qu'il consiste, essentiellement, à :

a/ sélectionner, ou à défaut fabriquer, des microparticules de PA de granulométrie comprise entre 50 et 1 000 microns, de préférence entre 100 et 750 microns et, plus préférentiellement encore, entre 100 et 500 microns,
b/ préparer la composition d'enrobage par mélange d'un polymère P1, d'un polymère P2, de l'agent plastifiant et de l'agent tensio-actif et/ou lubrifiant dans un système solvant,
c/ appliquer le mélange composition d'enrobage/système solvant sur des particules de PA,
d/ sécher les microcapsules ainsi obtenues,
e/ et éventuellement mélanger ces dernières avec au moins un agent anti-agglomérant.

9. Procédé selon la revendication 8, caractérisé en ce que le système solvant est formé par des composés sélectionnés dans la liste suivante : cétones, esters, solvants chlorés, alcools, de préférence aliphatiques, alcanes et leurs mélanges :

- les composés comptant de 1 à 6 carbones étant préférés,
- et l'acétone, la méthyléthylcétone, le méthanol, l'éthanol, l'isopropanol et le chlorure de méthylène étant particulièrement préférés.

10. Procédé selon la revendication 8 ou la revendication 9, caractérisé en ce que l'application du mélange composition d'enrobage/système solvant s'opère par pulvérisation sur les particules de PA mises en mouvement, de préférence par agitation mécanique ou par fluidisation.

11. Utilisation des microcapsules selon l'une quelconque des revendications 1 à 7 et/ou obtenues par le procédé selon l'une quelconque des revendications 8 à 10, pour la préparation de formes pharmaceutiques ou diététiques, de préférence sous forme de comprimés avantageusement délitables, de poudres ou de gélules.

12. Système galénique, de préférence sous forme de comprimé, avantageusement délitable, de poudre ou de gélule, caractérisé en ce qu'il comprend des microcapsules selon l'une quelconque des revendications 1 à 7 et/ou obtenues par le procédé selon l'une quelconque des revendications 8 à 10.

## Claims

1. Microcapsules of reservoir kind containing at least one medicinal and/or nutritional active principle (AP), with the exclusion of acetylsalicylic acid (ASA), which are intended for oral administration, characterized :

    - in that they consist of particles of AP each coated with one coating film of following composition :

        1- at least one film-forming polymer (P1) which is insoluble in the liquids of the digestive tract, present in a quantity of 50 to 90 %, preferably 50 to 80 % by weight of dry matter with respect to the total mass of the coating composition, and consisting of at least one non-hydrosoluble cellulose derivative, ethylcellulose and/or cellulose acetate being particularly preferred;
        2- at least one nitrogen-containing polymer (P2), present in a quantity of 2 to 25, preferably 5 to 15 % by weight of dry matter with respect to the total mass of the coating composition, and consisting of at least one polyacrylamide and/or one poly-N-vinylamide and/or one poly-N-vinyl-lactame, the polyacrylamide and/or the polyvinylpyrrolidone being particularly preferred;
        3- at least one plasticizer present in a quantity of 2 to 20 %, preferably 4 to 15 % by weight of dry matter with respect to the total mass of the coating composition, and consisting of at least one of the following compounds : glycerol esters, phtalates, citrates, sebacates, cetylalcohol esters, castor oil, salicyclic acid and cutin, castor oil being particularly preferred;
        4- at least one surface-active and/or lubricating agent, present in a quantity of 2 to 20 %, preferably 4 to 15 % by weight of dry matter with respect to the total mass of the coating composition, and chosen from anionic surfactants, preferably the alkali metal or alkakine-earth metal salts of fatty acids, stearic acid and/or oleic acid being preferred, and/or from nonionic surfactants, preferably polyoxyethylenated esters of sorbitan and/or polyoxyethylenated derivatives of castor oil, and/or from lubricants such as stearates, preferably calcium, magnesium, aluminium or zinc stearate, or such as stearylfumarate, preferably sodium stearylfumarate, and/or glyceryl behenate; said agent comprising only one or a mixture of the aforesaid products ;

    - in that they have a particle size of between 50 and 1 000 microns, preferably of between 100 and 750 microns and, more preferably, of between 100 and 500 microns ;
    - in that they are designed so as to be able to remain in the small intestine for a period of at least about 5 hours, preferably of at least about 7 hours and, even more preferably, for a period of between about 8 hours and about 24 hours, and permitting so the absorption of the AP during at least part of their residence in the small intestine.

2. Microcapsules according to Claim 1, characterized in that they comprise an amount of AP of between 55 and 95 % by weight, and preferably of between 60 and 85 % by weight.

3. Microcapsules according to Claim 1 or 2, characterized in that the composition of the coating film comprises from 60 to 80 % of ethylcellulose, from 5 to 10 % of polyvinylpyrrolidone, from 5 to 10 % of castor oil and from 2 to 8 % of magnesium stearate.

4. Microcapsules according to any one of Claims 1 to 3, characterized in that they are mixed with 0,5 to 5 % by weight, preferably 1,5 to 3 % by weight, of at least one anti-agglomerating agent formed, preferably, of talc, colloidal silica or of a mixture of the two.

5. Microcapsules according to any one of Claims 1 to 4, characterized in that the AP used belongs to at least one of the following families of active substances : antiulcer, antidiabetic, anticoagulant, antithrombic, hypolipaemic, antiarrhythmic, vasodilatory, antianginal, antihypertensive, and vasoprotective agents, fertility enhancers, labour inducers and inhibitors, and contraceptive, antibiotic, antifungal, antiviral, anticancer, anti-inflammatory, analgesic, antiepileptic, antiparkinsonian, neuroleptic, hypnotic, anxiolytic, psychostimulatory, antimigraine, antidepressant, antitussive, antihistamine or antiallergic agents.

6. Microcapsules according to Claim 5, characterized in that AP is chosen from the following compounds : pentoxifyllin, prazosin, acyclovir, nifedipin, diltiazem, naproxen, ibuprofen, flurbiprofen, ketoprofen, fenoprofen, indomethacin, diclofenac, fentiazac, oestradiol valerate, metoprolol, sulpiride, captopril, cimetidin, zidovudin, nicardipine, terfenadine, atenolol, salbutamol, carbamazepin, ranitidine, enalapril, simvastatin, fluoxetin, alprazolam, famotidin, ganciclovir, famciclovir, spironolacton, 5-asa, quinidin, perindopril, morphin, pentazocin, paracetamol, omeprazol, metoclopramid and mixtures thereof.

7. Microcapsules according to any one of Claims 1 to 6, characterized in that the AP consists of at least one nutritional and/or dietary supplement, preferably chosen from vitamins, amino acids, trace elements, antioxidants and mixtures thereof.

8. Process for producing the microcapsules according to any one of Claims 1 to 7, characterized in that it consists essentially in :

 a/ selecting, or in case of need making, microparticles of AP with a particle size of between 50 and 1 000 microns, preferably of between 100 and 750 microns and, even more preferably, of between 100 and 500 microns,
 b/ preparing the coating composition by mixing together a polymer P1, a polymer P2, the plasticizer and the surface active and/or lubricating agent in a solvent system,
 c/ applying the coating composition/solvent system mixture to particles of AP,
 d/ drying the microcapsules thus obtained, and
 e/ optionally, mixing these microcapsules with at least one anti-agglomerating agent.

9. Process according to Claim 8, characterized in that the solvent system is formed by compounds selected from the following list : ketones, esters, chlorinated solvents, alcohols, which are preferably aliphatic, alkanes and mixtures thereof :

- the compounds containing from 1 to 6 carbons being preferred, and

- acetone, methyl ethyl ketone, methanol, ethanol, isopropanol and methylene chloride being particularly preferred.

10. Process according to Claim 8 or Claim 9, characterized in that the coating composition/solvent system mixture is applied by spraying onto the particles of AP set in motion, preferably by mechanical stirring or by fluidization.

11. Use of microcapsules according to any one of Claims 1 to 7 and/or obtained by the method according to any one of Claims 8 to 10, for the preparation of pharmaceutical or dietetic forms, preferably in the form of tablets that can advantageously be crumbled, or powders or gelatin capsules.

12. Galenical system, preferably in the form of tablets that can advantageously be crumbled, or powders or gelatin capsules, characterised in that it comprises microcapsules according to any one of Claims 1 to 7 and/or obtained by the method according to any one of Claims 8 to 10.

**Patentansprüche**

1. Mikrokapseln vom Reservoirtyp, die mindestens einen Arzneimittelwirkstoff oder nutritionellen Wirkstoff (PA) mit der Ausnahme von Acetylsalicylsäure (ASA) enthalten und für die Verabreichung auf oralem Wege bestimmt sind, dadurch gekennzeichnet:

- daß sie aus PA-Teilchen gebildet werden, die jeweils bedeckt sind von einem dünnen Umhüllungsüberzug folgender Zusammensetzung:

 1. mindestens einem filmbildenden Polymer (P1), das in den Flüssigkeiten des Trakts unlöslich ist und in einer Menge von 50 bis 90, vorzugsweise 50 bis 80 % Trokkenmasse bezogen auf die gesamte Masse der Umhüllungszusammensetzung vorliegt und aus mindestens einem in Wasser unlöslichen Derivat von Cellulose gebildet wird, wobei Ethylcellulose und/oder Celluloseacetat besonders bevorzugt sind;
 2. mindestens einem stickstoffhaltigen Polymer (P2), das in einer Menge von 2 bis 25, vorzugsweise 5 bis 15 % Trockenmasse bezogen auf die gesamte Masse der Umhüllungszusammensetzung vorliegt und aus mindestens einem Polyacrylamid und/oder einem Poly-N-vinylamid und/oder einem Poly-N-vinyllactam gebildet wird, wobei Polyacrylamid und/oder Polyvinylpyrrolidon besonders bevorzugt sind;

3. mindestens einem Weichmacher, der in einer Menge von 2 bis 20, vorzugsweise 4 bis 15 % Trockenmasse bezogen auf die gesamte Masse der Umhüllungszusammensetzung vorliegt und aus mindestens einer der folgenden Verbindungen gebildet wird: den Glycerolestern, den Phthalaten, den Citraten, den Sebacaten, den Cetylalkoholestern, Rizinusöl, Salicylsäure und Cutin, wobei Rizinusöl besonders bevorzugt ist;

4. mindestens einem grenzflächenaktiven Mittel und/oder Gleitmittel, das in einer Menge von 2 bis 20, vorzugsweise 4 bis 15 % Trockenmasse bezogen auf die gesamte Masse der Umhüllungszusammensetzung vorliegt und ausgewählt ist aus den anionischen grenzflächenaktiven Mitteln, vorzugsweise den Alkali- oder Erdalkalimetallsalzen der Fettsäuren, wobei Stearinsäure und/oder Ölsäure bevorzugt sind, und/oder aus den nichtionischen grenzflächenaktiven Mitteln, vorzugsweise den Polyoxyethylen-Sorbitanestern und/oder den Derivaten von Polyoxyethylen-Rizinusöl und/oder unter den Gleitmitteln, wie den Stearaten, vorzugsweise von Calcium, Magnesium, Aluminium oder Zink, oder wie Stearylfumarat, vorzugsweise von Natrium, und/oder Glycerolbehenat; wobei das Mittel ein einzelnes oder eine Mischung der vorstehend genannten Produkte umfassen kann;

- daß sie eine Korngrößenverteilung zwischen 50 und 1000 μm, vorzugsweise zwischen 100 und 750 μm und noch mehr bevorzugt zwischen 100 und 500 μm aufweisen;

- daß sie so gestaltet sind, daß sie in dem Dünndarm während einer Zeitspanne von mindestens ungefähr 5 h, vorzugsweise von mindestens ungefähr 7 h und noch mehr bevorzugt während einer Zeitspanne zwischen ungefähr 8 h und ungefähr 24 h verweilen können und so die Absorption des PA während mindestens eines Teils ihrer Aufenthaltsdauer in dem Dünndarm ermöglichen.

2. Mikrokapseln nach Anspruch 1, dadurch gekennzeichnet, daß sie eine Menge an PA zwischen 55 und 95 Gew. -% und vorzugsweise zwischen 60 und 85 Gew. -% umfassen.

3. Mikrokapseln nach Anspruch 1 oder 2, dadurch gekennzeichnet, daß die Zusammensetzung des dünnen Umhüllungsüberzugs 60 bis 80% Ethylcellulose, 5 bis 10% Polyvinylpyrrolidon, 5 bis 10% Rizinusöl und 2 bis 8% Magnesiumstearat umfaßt.

4. Mikrokapseln nach einem der Ansprüche 1 bis 3, dadurch gekennzeichnet, daß sie mit 0,5 bis 5 Gew.-%, vorzugsweise 1,5 bis 3 Gew.-% von mindestens einem agglomerationsverhindernden Mittel, gebildet vorzugsweise aus Talk, kolloidalem Siliciumdioxid oder einer Mischung dieser beiden, vermischt sind.

5. Mikrokapseln nach einem der Ansprüche 1 bis 4, dadurch gekennzeichnet, daß der eingesetzte PA zu mindestens einer der folgenden Familien aktiver Substanzen gehört: Ulkustherapeutika, Antidiabetika, Antikoagulantien, Antithrombotika, Hypolipidämie-Therapeutika, Antiarrhythmika, Vasodilatatoren, antianginösen Mitteln, antihypertonischen Mitteln, Gefäßschutzmitteln, Fruchtbarkeitsförderern, Induktoren und Inhibitoren der Gebärmuttertätigkeit, Kontrazeptiva, Antibiotika, Fungiziden, Antivirusmitteln, Antikrebsmitteln, entzündungshemmenden Mitteln, Analgetika, Antiepileptika, Antiparkinsonmitteln, Neuroleptika, Hypnotika, Anxiolytika, Psychostimulantien, Antimigränemitteln, Antidepressiva, Antitussiva, Antihistaminika oder Antiallergika.

6. Mikrokapseln nach Anspruch 5, dadurch gekennzeichnet, daß der PA ausgewählt ist aus den folgenden Verbindungen: Pentoxifyllin, Prazosin, Acyclovir, Nifedipin, Diltiazem, Naproxen, Ibuprofen, Flurbiprofen, Ketoprofen, Fenoprofen, Indomethacin, Diclofenac, Fentiazac, Östradiolvalerat, Metoprolol, Sulpirid, Captopril, Cimetidin, Zidovudin, Nicardipin, Terfenadin, Atenolol, Salbutamol, Carbamazepin, Ranitidin, Enalapril, Simvastatin, Fluoxetin, Alprazolam, Famotidin, Ganciclovir, Famciclovir, Spironolacton, 5-asa, Quinidin, Perindopril, Morphin, Pentazocin, Paracetamol, Omeprazol, Metoclopramid und deren Mischungen.

7. Mikrokapseln nach einem der Ansprüche 1 bis 6, dadurch gekennzeichnet, daß der PA gebildet wird aus mindestens einer nutritionellen oder dietätischen Ergänzungssubstanz, vorzugsweise ausgewählt aus den Vitaminen, den Aminosäuren, den Oligoelementen, den Antioxidantien und deren Mischungen.

8. Verfahren zur Herstellung der Mikrokapseln nach einem der Ansprüche 1 bis 7, dadurch gekennzeichnet, daß es im wesentlichen daraus besteht:

a) PA-Mikropartikel mit einer Korngrößenverteilung zwischen 50 und 1000 μm, vorzugsweise zwischen 100 und 750 um und noch mehr bevorzugt zwischen 100 und 500 μm auszuwählen oder in Ermangelung derer herzustellen,

b) die Umhüllungszusammensetzung durch Mischen eines Polymers P1, eines Polymers P2, des Weichmachers und des grenzflächenaktiven Mittels und/oder Gleitmittels in einem Lösemittelsystem herzustellen,

c) die Mischung aus Umhüllungszusammensetzung und Lösemittelsystem auf die PA-Teilchen aufzubringen,

d) die so erhaltenen Mikrokapseln zu trocknen

e) und gegebenenfalls diese letzteren mit mindestens einem agglomerationsverhindernden Mittel zu mischen.

9. Verfahren nach Anspruch 8, dadurch gekennzeichnet, daß das Lösemittelsystem gebildet wird aus Verbindungen, ausgewählt aus der folgenden Liste: Ketonen, Estern, chlorierten Lösemitteln, Alkoholen, vorzugsweise aliphatischen, Alkanen und deren Mischungen:

- wobei die Verbindungen, die 1 bis 6 Kohlenstoffatome aufweisen, bevorzugt sind
- und Aceton, Methylethylketon, Methanol, Ethanol, Isopropanol und Methylenchlorid besonders bevorzugt sind.

10. Verfahren nach Anspruch 8 oder Anspruch 9, dadurch gekennzeichnet, daß das Aufbringen der Mischung aus Umhüllungszusammensetzung und Lösemittelsystem durch Zerstäuben auf den, vorzugsweise durch mechanische Bewegung oder durch Fluidisierung, in Bewegung gesetzten PA-Teilchen erfolgt.

11. Verwendung der Mikrokapseln nach einem der Ansprüche 1 bis 7 und/oder erhalten durch das Verfahren nach einem der Ansprüche 8 bis 10 für die Herstellung pharmazeutischer oder dietätischer Formen vorzugsweise in Form von vorteilhafterweise zerbröckelbaren Tabletten, Pulvern oder Kapseln.

12. Galenisches System, vorzugsweise in Form einer vorteilhafterweise zerbröckelbaren Tablette, eines Pulver oder einer Kapsel, dadurch gekennzeichnet, daß es Mikrokapseln nach einem der Ansprüche 1 bis 7 und/oder erhalten durch das Verfahren nach einem der Ansprüche 8 bis 10 enthält.

FIG 1

FIG 2

FIG 3

FIG4

FIG 5

FIG 6

(ng/ml)

FIG 7

(%. abs)

FIG 8